**Europäisches Patentamt**

**European Patent Office**

**Office européen des brevets**

(19)

(11) Veröffentlichungsnummer: **0 195 943**

**B1**

(12)

# EUROPÄISCHE PATENTSCHRIFT

(45) Veröffentlichungstag der Patentschrift: **10.10.90**

(21) Anmeldenummer: **86102424.8**

(22) Anmeldetag: **25.02.86**

(51) Int. Cl.⁵: **C 07 C 29/60,** C 07 C 33/025, B 01 J 27/18

(54) **Katalysatorsystem und Verfahren zur Herstellung von alpha,omega-C4- bis C20-Alkenolen.**

(30) Priorität: **23.03.85 DE 3510568**

(43) Veröffentlichungstag der Anmeldung:
**01.10.86 Patentblatt 86/40**

(45) Bekanntmachung des Hinweises auf die Patenterteilung:
**10.10.90 Patentblatt 90/41**

(84) Benannte Vertragsstaaten:
**BE CH DE FR GB IT LI NL**

(56) Entgegenhaltungen:
**EP-A-0 015 318**
**FR-A-2 262 005**
**GB-A-1 355 704**

(73) Patentinhaber: **HÜLS AKTIENGESELLSCHAFT Patentabteilung / PB 15 - Postfach 13 20 D-4370 Marl 1 (DE)**

(72) Erfinder: **Kleine-Homann, Walter, Dr. Buchenallee 14 D-4408 Dülmen (DE)** Erfinder: **Fischer, Lothar, Dr. Leverkusener Strasse 15 D-4370 Marl (DE)**

Courier Press, Leamington Spa, England.

EP 0 195 943 B1

EP 0 195 943 B1

## Beschreibung

Zur Synthese von α,ω-C₄- bis C₂₀-Alkenolen mit Reinheitsgraden über 85% durch katalytische Dehydratisierung der entsprechenden α,ω-C₄- bis C₂₀-Diole setzt man nach dem Verfahren der DE-PS 29 04 518 neutrale, einfache oder Misch-Pyrophosphate des Lithiums, Natriums, Strontiums oder Bariums bzw. Gemische dieser Verbindungen als Katalysator ein. Der Nachteil dieses Verfahrens liegt zum einen in der Bildung merklicher Anteile von Alkanolen, zum anderen steigt die Bildung anderer Nebenprodukte bei Umsätzen von >80% merklich und bei Umsätzen von ≧90% erheblich an (Spalte 3, Zeilen 43 bis 47). Hieraus ergab sich die Aufgabe, ein Verfahren zu finden, das neben der Reduzierung des Alkanolgehalts auch bei Umsätzen über 90% eine noch genügend hohe Selektivität aufweist.

Diese Aufgabe wird erfindungsgemäß entsprechend den Angaben der Patentansprüche gelöst.

Überraschenderweise zeigen die beanspruchten Katalysatoren, wie sie beispielsweise nach den Angaben der Beispiele hergestellt wurden, im Gegensatz zur Lehre der DE-PS 29 04 518 auch bei Umsätzen von über 90% hohe Selektivitäten. Dies ermöglicht eine wirtschaftlichere Herstellung von Produkten mit Reinheitsgraden über 90% So beträgt der Alkanolanteil lediglich 0,3 bis 4,5%, vorzugsweise 0,5 bis 2,5%, im Rohaustrag bei einem gleichzeitigen Gehalt an Diolefinen von 0,3 bis 7,0%, vorzugsweise von 0,5 bis 5,0%. Zudem werden mit dem erfindungsgemäßen Katalysatorsystem erheblich verlängerte Standzeiten von mehr als 5 Monaten erzielt, ohne daß ein nennenswerter Aktivitäts- und Selektivitätsverlust festgestellt wurde. Dies war nicht zu erwarten, da nach Chem. Abstr. *58*, (1963), 2353 ein mit einer basischen Verbindung wie Natronlauge behandeltes Tricalciumphosphat als Dehydratisierungskatalysator bereits nach 100 h regeneriert werden muß, da seine Aktivität zu stark abgenommen hat.

Als Additive eignen sich Alkali- und Erdalkaliverbindungen, beispielsweise die Oxide, Hydroxide, Carbonate oder Phosphate von Metallen der 1. Hauptgruppe und/oder der 2. Hauptgruppe wie des Beryllium, Magnesium, Calzium und/oder Strontium. Bevorzugt setzt man die Phosphate als Diphosphate ein, insbesondere die des Natriums. Die Additive gibt man vorzugsweise in Mengen von 2 bis 25%, insbesondere von 5 bis 15%, berechnet als Metalloxid, bezogen auf die Hauptkomponente (berechnet als Metalloxid), zu.

Als Hauptkomponente des Katalysators setzt man einfache oder Mischphosphate der Elemente der 2. Hauptgruppe ein, wie beispielsweise Barium-ortho-phosphat oder Bariumhydrogenphosphat. Bevorzugt setzt man die Bariumverbindungen ein. Man kann natürlich auch Oxide, Hydroxide oder Salze wie z. B. Carbonate des Bariums mit Phosphorsäure umsetzen und das Umsetzungsprodukt als Hauptkomponente einsetzen. Diese Phosphate aktiviert man mit den Additiven.

Die zum Stande der Technik gehörenden Katalysatoren (z. B. DE-PS 21 17 444 und DE-PS 29 04 518) werden hergestellt durch Fällung aus wäßrigen Lösungen von beispielsweise Natriumdiphosphat mit Bariumnitrat. Diese Herstellungsweise erfordert jedoch die Handhabung größerer Volumina an Lösungen, Mutterlaugen und Waschwasser, die man mit großen Aufwand reinigen muß, ehe man sie, ohne die Umwelt zu belasten, ins Abwasser geben kann. Hinzukommt die schwierige Einstellung des Natriumsalzgehaltes bei der Katalysatorherstellung durch Fällung.

Die erfindungsgemäße Umstellung der Präparation auf eine Reaktion zwischen einer Erdalkaliverbindung, beispielsweise Bariumcarbonat und Phosphorsäure mit nachfolgendem definiertem Zusatz einer Alkali- oder Erdalkaliverbindung, beispielsweise von Natriumdiphosphat, bringt eine wesentliche Verbesserung, wobei die Zugabe des Natriumdiphosphates in ganz bestimmter Weise mit niedriger Dosiergeschwindigkeit zu erfolgen hat, um sofort eine verformbare Masse zu erhalten.

Obwohl nach einer Calcinierung von 350°C bis 950°C, z. B. die Wasserabspaltung aus einem vorher gebildeten Bariumhydrogenphosphat die Bildung von Bariumdiphosphat neben dem Natriumdiphosphat bedingen sollte, zeigt das Röntgenstrahlbeugungsdiagramm Reflexe, die nicht den in der JCPDS-Kartei für Bariumdiphosphat registrierten entsprechen. Hingegen finden sich IR-Absorptionsspektrum Banden, die mit einer Diphosphatstruktur verträglich sind.

Die aktivsten und selektivsten Katalysatoren wurden dann erhalten, wenn zum Erdalkaliphosphat bzw. — wie oben geschildert — nach einer vollständigen Umsetzung des Erdalkalicarbonates mit der Phosphorsäure die Alkali- oder Erdalkaliverbindung, wie z. B. das Natriumdiphosphat, mit kleiner Dosiergeschwindigkeit zugemischt wurde. Beispielsweise beträgt die Dosiergeschwindigkeit 5 g/h bis 20 g/h bei einem Einsatz von 1 bis 10 kg Bariumcarbonat, damit die Reaktionstemperatur 30°C nicht übersteigt. Der so nach Calcinierung von 350 bis 950°C erhaltene Katalysator zeichnet sich im IR-Absorptionsspektrum dadurch aus, daß bei 700 bis 720 cm⁻¹, bei 750 cm⁻¹ und bei 1 110 cm⁻¹ keine bzw. nur sehr schwache Banden und bei 920 bis 924 cm⁻¹ und 1 126 bis 1 130 sehr starke Banden auftreten.

Die erfindungsgemäßen Katalysatorsysteme stellt man her, indem man z. B. das Bariumcarbonat mit ortho-Phosphorsäure versetzt und danach die erforderliche Menge des Additivs zusetzt. Die erhaltene Masse wird getrocknet, extrudiert und bei 350°C bis 950°C, bevorzugt bei 400°C bis 600°C, calciniert, wie u. a. in den Beispielen ausführlich beschrieben.

Die Strukturen der erfindungsgemäßen Katalysatoren sind noch unbekannt.

Die IR-Absorptionsspektren und Röntgenstrahlbeugungsdiagramme der katalysatoren 2 und 3 weichen beispielsweise von denen der Di-phosphate ab (s. Beispiele 2b und 3b). Somit handelt es sich sowohl nach der Herstellung als auch nach den IR-Spektren und Röntgenstrahlbeugungsdiagrammen um einen anderen Katalysator als den in der DE-PS 29 04 518 beanspruchten (Di- bzw. Pyrophosphatkatalysator).

2

Die Dehydratisierung der Edukte erfolgt bei Temperaturen von 300°C bis 500°C, vorzugsweise 350°C bis 450°C. Im allgemeinen arbeitet man bei Normaldruck, man kann aber auch bei Unter- oder Überdruck arbeiten. Die Umsetzung erfolgt vorzugsweise kontinuierlich, man kann aber auch diskontinuierlich arbeiten.

Zur Steigerung der Selektivität ist es empfehlenswert, das Edukt mit Inerten wie beispielsweise Stickstoff, Edelgasen oder Wasserdampf in der Reaktions- oder Verdampfungszone abzumischen. Gleichzeitig geht damit eine Verlängerung der katalysatorstandzeit einher. Im allgemeinen setzt man die Inerten in Mengen von 1 bis 90 Mol-% ein, bevorzugt von 25 bis 60 Mol-%.

Nach dem erfindungsgemäßen Verfahren erhält man die α,ω-Alkenole auch bei Umsätzen über 90% mit hoher Selektivität (70 bis 95%) und in hoher Reinheit (über 90%). Die erhaltenen α,ω-Alkenole mit 4 bis 20 C-Atomen sind wertvolle Riechstoffe und Zwischenprodukte für zahlreiche weitere technische Synthesen.

Vergleichsbeispiel A 1

Herstellung des Katalysators A
(ohne Aktivierung mit Alkali- oder Erdalkaliverbindung)

Sulfidfreies Bariumcarbonat wird bei ca. 40°C im Kneter mit wäßriger Phosphorsäurelösung umgesetzt. Das Reaktionsprodukt, $BaHPO_4$, wird vom überschüssigen Wasser soweit befreit, daß eine extrudierbare Mischung entsteht. Diese wird extrudiert und anschließend bis 450°C calciniert.

In einem Kneter werden 4 000 g sulfidfreies Bariumcarbonat (Lieferant: Kali-Chemie) bei hoher Drehgeschwindigkeit der Knetarme bei ca. 40°C mit 2 348 g wäßriger 84,6 %iger Phosphorsäurelösung in 77 min versetzt. Es wird 1 h bei 40°C und anschließend ca. 0,5 h bei 60°C Produkttemperatur weiter geknetet. Man erhält ca. 5 260 g einer Masse, die mit einem Extruder (Fabrikat: Hutt-Bepex) zu 4 mm-Stränge verformt wird. Die Extrudate werden in einem Schachtofen im Luftstrom bei 110°C getrocknet, wobei ein Masseverlust von ca. 8,7% auftritt. Die getrockneten Stränge werden anschließend in einem Schachtofen im Luftstrom in etwa 2,2 h von 110 auf 450°C erhitzt und calciniert und 4 h bei dieser Temperatur gehalten. Es tritt ein Masseverlust von ca. 4,7% auf, wobei man einen Katalysator mit folgenden Eigenschaften erhält:

Chemische Zusammensetzung:

| | |
|---|---|
| BaO-Gehalt | 66,1% |
| SrO-Gehalt | 1,0% |
| $P_2O_5$-Gehalt | 32,2% |
| $CO_2$-Gehalt | 0,34% |
| $SO_3$-Gehalt | 0,11% |
| Struktur: | Das Röntgenstrahlbeugungsdiagramm ergibt keinen eindeutigen Hinweis, daß $Ba_2P_2O_7$ vorliegt, das IR-Spektrum enthält Banden, die dem $Ba_2P_2O_7$ zuzuordnen sind. |

Physikalische Eigenschaften:

| | |
|---|---|
| Form | Stränge |
| Durchmesser | 4 mm |
| Schüttdichte | 1,69 /cm³ |
| spez. Porenvolumen | |
| (gesamt) | 0,16 cm³/g |
| ($D_P$ >8 nm) | 0,12 cm³/g |
| spez. innere Oberfläche | |
| ($D_P$ >8 nm) | 4,5 m²/g |

Röntgenstrahlbeugungsdiagramm und IR-Absorptionsspektrum s. S. 7 und 8.

Vergleichsbeispiel B 1

Herstellung des Katalysators B

Eine Probe des nach den Angaben des Vergleichsbeispiels A 1 hergestellten und bei 110°C getrockneten Katalysators wird anschließend bei 950°C 1 h im Muffelofen behandelt. Danach wird sie, da sie zusammengesintert ist, zerkleinert und zur Testung gegeben. Das RSB-Diagramm dieses Materials ist mit dem in der JCPDS-Kartei unter Nr. 30—144 geführten $Ba_2P_2O_7$ identisch (siehe Diagramm Katalysator B 1). Hingegen enthält das IR-Absorptionsspektrum zusätzlich zu den von M. Watonabe et al., Bull. Chem. Soc. Jpn. *56* (1983), 3430 veröffentlichten Banden weitere, deren Zuordnung unbekannt ist.

Röntgenstrahlbeugungsdiagramm und IR-Absorptionsspektrum s. S. 7 und 8.

# EP 0 195 943 B1

### Röntgenstrahlbeugungsdiagramm — Katalysator A

| 2 θ (°) | d (nm) | I (%) |
|---|---|---|
| 11,5 | 0,769 | 23 |
| 12,9 | 0,686 | 8 |
| 19,2 | 0,462 | 8 |
| 25,0 | 0,356 | 100 |
| 25,8 | 0,345 | 11 |
| 27,3 | 0,326 | 31 |
| 28,1 | 0,317 | 9 |
| 29,6 | 0,302 | 14 |
| 31,8 | 0,281 | 38 |
| 38,8 | 0,232 | 8 |
| 39,2 | 0,230 | 15 |
| 42,4 | 0,213 | 40 |
| 47,0 | 0,193 | 17 |
| 52,8 | 0,173 | 9 |

θ: Beugungswinkel
d: Netzebenenabstand
I: Intensität

IR-Absorptionsspektrum - Katalysator A

# EP 0 195 943 B1

Katalysator B
Röntgenstrahlbeugungsdiagramm

I/(%)

2 θ/(°)

| 2 θ (°) | d (nm) | I (%) |
|---|---|---|
| 22,7 | 0,391 | 100 |
| 23,0 | 0,386 | 33 |
| 25,1 | 0,354 | 28 |
| 29,0 | 0,307 | 11 |
| 31,3 | 0,285 | 44 |
| 32,9 | 0,272 | 36 |
| 38,4 | 0,232 | 27 |
| 39,6 | 0,227 | 37 |
| 40,2 | 0,224 | 24 |
| 41,6 | 0,217 | 23 |
| 41,7 | 0,216 | 22 |
| 42,4 | 0,213 | 26 |
| 42,6 | 0,212 | 22 |
| 46,0 | 0,197 | 23 |
| 46,1 | 0,197 | 18 |
| 50,1 | 0,182 | 16 |

θ: Beugungswinkel
d: Netzebenenabstand
I: Intensität

IR-Absorptionsspektrum - Katalysator B

EP 0 195 943 B1

# EP 0 195 943 B1

## Vergleichsbeispiel C 1

### Herstellung des Vergleichskatalysators gemäß DE-PS 21 17 444

In wäßriger Lösung wird Bariumdiphosphat durch Zusammengeben wäßriger Lösungen von Natriumdiphosphat zu Bariumnitrat gefällt. Die überstehende Lösung kann man dekantieren und den Rest filtrieren. Man kann auch die Suspension insgesamt filtrieren. Nach Teiltrocknung der Masse wird sie extrudiert. Die Stränge werden bei 110°C getrocknet und bei 550°C calciniert.

Es werden folgende Lösungen hergestellt:

1. Wässrige, 22,78 %ige $Na_4P_2O_7$-Lösung, durch Auflösen von 88,5 kg (0,3328 mol) wasserfreiem $Na_4P_2O_7$ in 300 kg destilliertem Wasser bei 90°C.

2. Wässrige, 11,42 %ige $Ba(NO_3)_2$-Lösung, durch Auflösen von 174 kg (0,6656 mol) $Ba(NO_3)_2$ in 1 350 kg destilliertem Wasser bei 55°C.

Die wässrige $Ba(NO_3)_2$-Lösung wird unter Rühren innerhalb von 45 min in die $Na_4P_2O_7$-Lösung gegeben. Es wird 3 h weiter gerührt. Temperatur: ca. 50°C, pH-Wert: 11,0. Danach wird die Suspension filtriert, der Filterkuchen wird, ohne ihn vorher zu waschen, bei 50°C auf einem Gehalt an Feuchtigkeit von (30 ... 33)% getrocknet. Nach Zerkleinern wird die Masse zu 6 mm-Strängen extrudiert, die Stränge werden in Luft bei 110°C getrocknet und anschließend bei 550°C calciniert.

Es werden 132 kg (=88,4% d. Th) erhalten. Der katalysator hat folgende Eigenschaften:

| | |
|---|---|
| BaO-Gehalt | 64,5% |
| $P_2O_5$-Gehalt | 31,2% |
| $Na_2O$-Gehalt | 3,6% |
| $NO_3$-Gehalt | <0,1% |
| Glühverlust (bei 850°C) | 0,1% |
| | |
| Form | Stränge |
| Durchmesser | 6 mm |
| Schüttdichte | 1,23 g/cm³ |
| spez. Porenvolumen | 0,23 cm³/g |
| spez. innere Oberfläche | 1,2 m²/g |

## Beispiel 1a

### Herstellung des Katalysators I

400 g sulfidfreies Bariumcarbonat werden bei Raumtemperatur innerhalb von 30 min mit 260 g 75 %iger wäßriger Phosphorsäure möglichst intensiv angeteigt und sodann unter weiterem Vermischen mit 90 g Tetranatriumdiphosphat versetzt. Der gebildete Salzteig wird danach vorsichtig im Stickstoffstrom über 12 h im Vakuum bei ca. 100°C getrocknet.

Die so erhärtete Salzmasse wird mechanisch in kleine unregelmäßige Bruchstücke von etwa 1 bis 10 mm zerkleinert und innerhalb von 24 h bei 550°C im Stickstoffstrom calciniert.

Der Natriumgehalt im calcinierten Produkt beträgt 5,2%.

## Beispiel 2a

### Katalysator II

In einem Kneter werden 395 g sulfidfreies Bariumcarbonat (Lieferant: Kali-Chemie) bei Raumtemperatur in 17 min mit 256 g 76,6 %iger wäßriger Phosphorsäurelösung versetzt, wobei die Temperatur um 5 bis 10°C ansteigt. Nach 10 minütigem Nachkneten werden 183 g Tetranatriumdiphosphat zugegeben. Es wird 10 min weiter geknetet. Aus dem Kneter werden erhalten: 720 g (berechnet aus Einsatzstoffmengen: 746 g).

Die Mischung wird zum Verformen durch einen Extruder (Fabrikat: Hutt) gegeben. Die entstandenen 4 mm-Stränge werden im Luftstrom 19 h bei 110°C getrocknet und danach 19 h bei 500°C calciniert.

Masseverlust beim Trocknen: 9,7%

Masseverlust beim Calcinieren: 5,8%

8

**EP 0 195 943 B1**

Chemische Zusammensetzung
BaO-Gehalt:                          47,6%

Na$_2$O-Gehalt:                      11,5%

P$_2$O$_5$-Gehalt:                   37,0%

Physikalische Eigenschaften
Form                                 Stränge

Durchmesser                          4 mm

Länge                                2 ... 10 mm

Schüttdichte                         1,27 g/cm$^3$

spezif. Porenvolumen                 0,17 cm$^3$/g

spezif. innere Oberfläche            0,8 m$^2$/g

spezif. Seitendruckfestigkeit        18 ± 8 N/mm

Katalysator II
Röntgenstrahlbeugungsdiagramm

| 2 θ (°) | d (nm) | I (%) |
|---|---|---|
| 11,5 | 1,768 | 27 |
| 13,0 | 1,682 | 12 |
| 19,2 | 1,462 | 12 |
| 20,2 | 1,440 | 13 |
| 25,1 | 1,354 | 100 |
| 28,2 | 1,316 | 12 |
| 26,7 | 1,301 | 20 |
| 32,0 | 1,279 | 40 |
| 38,9 | 1,231 | 13 |
| 39,4 | 1,229 | 21 |
| 42,6 | 1,212 | 53 |
| 46,9 | 1,194 | 19 |
| 47,2 | 1,193 | 25 |
| 52,9 | 1,173 | 14 |

θ: Beugungswinkel
d: Netzebenenabstand
I: Intensität

9

IR-Absorptionsspektrum - Katalysator II

Beispiel 3a, Katalysator III

Herstellung eines Dehydratisierkatalysators mit Ba-Diphosphat als Hauptbestandteil

In einem Kneter werden 3 000 g sulfidfreies Bariumcarbonat (Lieferant: Kali-Chemie) bei einer hohen Drehgeschwindigkeit der Knetarme von 81 und 135 min$^{-1}$ bei 40°C mit 1 761 g 84,6 %iger wäßriger Phosphorsäurelösung in 73 min versetzt. Es wird noch 30 min weitergeknetet und anschließend in 27 min 555 g destilliertes Wasser zugegeben. Darauf wird 1 386 g Tetranatriumdiphosphat in einer Portion unter Kneten zugefügt, wobei die Temperatur schnell auf 55°C ansteigt und danach langsam abfällt. Es wird 40 min gemischt. Es entsteht ein Pulver, das viele gröbere Granulate enthält. Menge ca. 5 900 g (berechnet aus Einsatzstoffen unter Abzug der Menge entwickelten $CO_2$: 6 033 g).

Zum Extrudieren wird nochmals Wasser im Verhältnis 1:26 zum Produkt hinzugesetzt und die so erhaltene Mischung bei 50°C zu 4 mm-Strängen verformt. Die Stränge werden im Luftstrom zuerst 16 h bei 110°C getrocknet und dann 4 h bei 450°C calciniert.

Beim Trocknen verliert die Masse ca. 19% an Gewicht und beim Calcinieren 3,7%.

Chemische Zusammensetzung

| | |
|---|---|
| BaO-Gehalt | 46,6% |
| SrO-Gehalt: | 0,7% |
| $Na_2O$-Gehalt: | 13,3% |
| $P_2O_5$-Gehalt: | 38,8% |

Struktur

| | |
|---|---|
| per Röntgenstrahlbeugung: | $Na_4P_2O_7$ nachgewiesen; weitere Reflexe gemäß JCPDS-Kartei nicht zuzuordnen |
| per IR-Spektroskopie: | die Absorptionsbanden bei 735 cm$^{-1}$ (P—O—P-Biegungsschwingung) und bei 921 und 956 cm$^{-1}$ (P—O-Streckschwingung) deuten auf Diphosphat hin. |

Physikalische Eigenschaften

| | |
|---|---|
| Form | Stränge |
| Durchmesser | 4 mm |
| Schüttdichte | 1,69 g/cm$^3$ |
| spezif. Porenvolumen | |
| (gesamt) | 0,25 cm$^3$/g |
| ($D_P$ >56 nm) | 0,21 cm$^3$/g |
| spezif. innere Oberfläche | |
| (gesamt) | 3 m$^2$/g |
| ($D_P$ >56 nm) | 1,7 m$^2$/g |

Katalysator III
Röntgenstrahlbeugungsdiagramm

| 2 θ (°) | d (nm) | I (%) |
|---|---|---|
| 11,6 | 0,762 | 22 |
| 13,0 | 0,680 | 12 |
| 19,4 | 0,457 | 11 |
| 20,3 | 0,441 | 22 |
| 25,2 | 0,353 | 100 |
| 25,8 | 0,345 | 14 |
| 27,5 | 0,324 | 29 |
| 28,3 | 0,315 | 10 |
| 29,7 | 0,300 | 12 |
| 32,1 | 0,278 | 41 |
| 32,8 | 0,273 | 10 |
| 33,3 | 0,269 | 19 |
| 34,0 | 0,263 | 11 |
| 36,0 | 0,249 | 9 |
| 39,0 | 0,231 | 9 |
| 39,5 | 0,228 | 13 |
| 42,6 | 0,212 | 42 |
| 47,1 | 0,193 | 20 |
| 51,7 | 0,176 | 7 |
| 53,0 | 0,173 | 12 |

θ: Beugungswinkel
d: Netzebenenabstand
I: Intensität

IR-Absorptionsspektrum - Katalysator III

EP 0 195 943 B1

Beispiele 1b, 2b, 3b, 4 und 6 zur Herstellung der α,ω-Enole

1 l des entsprechenden Kontaktes (s. Tabelle) wird in einem beheizbaren Quarzrohr von 1 m Länge und 50 mm Durchmesser vorgelegt. Unter Zugabe von Stickstoff wird der jeweilige Kontakt auf die gewünschte Temperatur (s. Tabelle) erwärmt. Die Temperaturerfassung erfolgt mit Hilfe eines Widerstandsthermometers, das sich in einem zentrierten Quarzrohr von 6 mm Durchmesser befindet und sich dort je nach Anforderung in die gewünschte Position axial verschieben läßt. Das geschmolzene α,ω-Alkandiol wird sodann über einen Verdampfer mit nachgeschaltetem Überhitzer auf die gewünschte Reaktionstemperatur erhitzt und mit den Inerten auf den Katalysator gegen. Der kondensierte Reaktoraustrag wird anhand einer GC- und Wasseranalyse untersucht. Die Bestätigung dieser Werte erfolgt durch Fraktionierung des Rohproduktes im Vakuum. Die Destillation wird an einer 0,5 m-Mulfifilkolonne vorgenommen. Nach Abnahme von Wasser und Leichtsiedern im Normaldruck wird der Hauptlauf bei einem Vakuum von ca. 10 mbar isoliert.

| Beispiel | Edukt | Produkt | Kontakt | Kontakt-temp. (°C) | LHSV ml/ml h | Umsatz | Alkanol-gehalt (%) | Dien-gehalt (%) | Selekti-vität (%) | Rein-heit(%) |
|---|---|---|---|---|---|---|---|---|---|---|
| Vergleichsbei-spiele |  |  |  |  |  |  |  |  |  |  |
| A 2 | Decandiol | Decenol | A | 430 | 0,30 | 68 | 6,8 | 8,3 | 64,7 | 91,9 |
| B 2 | Hexandiol | Hexenol | B | 380 | 0,29 | 94,8 | 0,5 | 11,9 | 49,5 | 84,9 |
| C 2 | Hexandiol | Hexenol | C | 400 | 0,45 | 49,1 | 7,4 | 13,5 | 55,0 | 82,7 |
| Beispiele |  |  |  |  |  |  |  |  |  |  |
| 1 b | Decandiol | Decenol | I | 400 | 0,45 | 67,8 | 4,4 | 6,9 | 73,5 | 93,2 |
| 2 b | Decandiol | Decenol | II | 430 | 0,29 | 94,0 | 0,8 | 5,8 | 84,1 | 95,8 |
| 3 b | Decandiol | Decenol | III | 410 | 0,30 | 83,4 | 0,7 | 1,6 | 87,8 | 96,5 |
| 4 | Octandiol | Octenol | II | 420 | 0,29 | 94,9 | 1,4 | 1,9 | 85,8 | 95,4 |
| 5 | Hexandiol | Hexenol | II | 440 | 0,25 | 96,2 | 1,9 | 6,4 | 70,0 | 92,8 |

# EP 0 195 943 B1

**Patentansprüche**

**Patentansprüche**

1. Verfahren zur Herstellung von reinen $\alpha,\omega$-$C_4$- bis $C_{20}$-Alkenolen mit Reinheitsgraden über 90% durch katalytische Dehydratisierung der entsprechenden $\alpha,\omega$-$C_4$- bis $C_{20}$-Diole, dadurch gekennzeichnet, daß man $\alpha,\omega$-$C_4$- bis $C_{20}$-Diole mit Hilfe von mit Alkali- oder Erdalkali-Verbindungen dotierten einfachen oder Misch-Phosphaten der Elemente der 2. Hauptgruppe als Katalysatoren, wobei man durch Einsatz von Erdalkaliortho-phosphat oder Erdalkalihydrogenphosphat bzw. durch Umsetzung einer Erdalkaliverbindung mit Phosphorsäure zum entsprechenden Phosphat und Zusatz einer Alkali- oder Erdalkaliverbindung eine Katalysatormasse herstellt, diese gegebenenfalls verformt, anschließend trocknet und sie dann bei Temperaturen von 350 bis 950°C calciniert, bei Temperaturen von 300 bis 500°C selektiv und partielle zu $\alpha,\omega$-$C_4$- bis $C_{20}$-Alkenolen mit Reinheitsgraden über 90% bei Umsätzen von über 90% dehydratisiert.

2. Verfahren nach Anspruch 1, dadurch gekennzeichnet, daß man als Erdalkaliverbindung eine Bariumverbindung einsetzt.

3. Verfahren nach Anspruch 1 und 2, dadurch gekennzeichnet, daß man als Alkali- oder Erdalkaliverbindung die Carbonate, Oxide, Hydroxide und/oder Phosphate dieser Metalle in Mengen von 2 bis 25%, bevorzugt 5 bis 15%, berechnet als Metalloxid und bezogen auf das Erdalkaliphosphat, berechnet als Erdalkalioxid, einsetzt.

4. Verfahren nach den Ansprüchen 1 bis 3, dadurch gekennzeichnet, daß man als Alkali- oder Erdalkaliverbindung die Carbonate, Oxide, Hydroxide und/oder Phosphate des Natriums einsetzt.

5. Verfahren nach den Ansprüchen 1 bis 4, dadurch gekennzeichnet, daß man den Katalysator bei Temperaturen von 400 bis 600°C calciniert.

6. Verfahren nach den Ansprüchen 1 bis 5, dadurch gekennzeichnet, daß man das Edukt bei Temperaturen von 350 bis 450°C dehydratisiert.

**Revendications**

1. Procédé de préparation à l'état pur d'alpha,oméga-alcénols en $C_4$ à $C_{20}$ présentant des degrés de pureté supérieurs à 90%, par déshydratation catalytique des alpha,oméga-diols en $C_4$ à $C_{20}$ correspondants, caractérisé par le fait qu'à des températures de 300 à 500°C, on déshydrate sélectivement et partiellement en alpha,oméga-alcénols en $C_4$ à $C_{20}$ présentant des degrés de pureté supérieurs à 90%, et avec des rendements supérieurs à 90%, des alpha,oméga-diols en $C_4$ à $C_{20}$ à l'aide de phosphates simples ou mixtes (dotés de composés alcalins ou alcalino-terreux) des éléments du deuxième groupe principal en tant que catalyseurs, tandis qu'en utilisant un ortho-phosphate de métal alcalino-terreux ou un hydrogéno-phosphate de métal alcalino-terreux ou en faisant réagir un composé alcalino-terreux sur de l'acide phosphorique pour obtenir le phosphate correspondant et qu'en ajoutant un composé alcalin ou alcalino-terreux, on prépare une masse de catalyse, que l'on façonne éventuellement celle-ci, qu'on la sèche ensuite et qu'on la calcine alors à des températures de 350 à 950°C.

2. Procédé selon la revendication 1, caractérisé par le fait que l'on utilise, comme composé alcalino-terreux, un composé de baryum.

3. Procédé selon la revendications 1 et 2, caractérisé par le fait que l'on utilise, comme composé alcalin ou alcalino-terreux, les carbonates, oxydes, hydroxydes et/ou phosphates de ces métaux dans des quantités de 2 à 25% de préférence de 5 à 15%, calculées en oxyde métallique et relativement au phosphate alcalino-terreux, calculé en tant qu'oxyde alcalino-terreux.

4. Procédé selon les revendications 1 à 3, caractérisé par le fait que l'on utilise, comme composé alcalin ou alcalino-terreux, les carbonates, oxydes, hydroxydes et/ou phosphates du sodium.

5. Procédé selon les revendications 1 à 4, caractérisé par le fait que l'on calcine le catalyseur à des températures de 400 à 600°C.

6. Procédé selon les revendications 1 à 5, caractérisé par le fait que l'on déshydrate le produit formé à des températures de 350 à 450°C.

**Claims**

1. A process for the preparation of a pure $\alpha,\omega$-$C_4$- to $C_{20}$-alkenol having a degree of purity of greater than 90% by catalytic dehydration of the corresponding $\alpha,\omega$-$C_4$- to $C_{20}$-diol, characterized in that a $\alpha,\omega$-$C_4$- to $C_{20}$-diol is selectively and partially dehydrated at a temperature of from 300 to 500°C to give an $\alpha,\omega$-$C_4$- to $C_{20}$-alkenol having a degree of purity of greater than 90% with a conversion of greater than 90% with the aid of, as catalyst, a simple or mixed phosphate of an element of main group 2 which has been doped with an alkali metal compound or alkaline earth metal compound, a catalyst composition being prepared by employing an alkaline earth metal orthophosphate or alkaline earth metal hydrogen phosphate or by reacting an alkaline earth metal compound with phosphoric acid to give the corresponding phosphate and adding an alkali metal compound or alkaline earth metal compound, the catalyst composition being shaped if desired, subsequently dried and then calcined at a temperature of from 350 to 950°C.

2. A process according to claim 1, characterized in that, as the alkaline earth metal compound, a barium compound is employed.

3. A process according to claim 1 or 2, characterized in that, as the alkali metal compound or alkaline

16

earth metal compound, a carbonate, oxide, hydroxide and/or phosphate of these metals is employed in an amount of from 2 to 25%, preferably 5 to 15%, calculated as the metal oxide and relative to the alkaline earth metal phosphate, calculated as the alkaline earth metal oxide.

4. A process according to any of claims 1 to 3, characterized in that, as the alkali metal compound or alkaline earth metal compound, a carbonate, oxide, hydroxide and/or phosphate of sodium is employed.

5. A process according to any of claims 1 to 4, characterized in that the catalyst is calcined at a temperature of from 400 to 600°C.

6. A process according to any of claims 1 to 5, characterized in that the educt is dehydrated at a temperature of from 350 to 450°C.